# EUROPEAN PATENT APPLICATION

(11) **EP 2 181 657 A2**
(43) Date of publication of application: **05.05.2010**
(21) Application number: 09252508.8
(22) Date of filing: 29.10.2009
(51) Int. Cl.: A61B 17/34

(54) **Surgical access port with adjustable ring geometry**

(30) Priority: 30.10.2008 US 261696
(71) Applicant: Ethicon Endo-Surgery, Inc., Cincinnati, OH 45242 (US)
(72) Inventor: Widenhouse, Christopher W., Clarksville Ohio 45113 (US); Shelton, Frederick E., IV., Hillsboro Ohio 45133 (US)
(74) Representative: Tunstall, Christopher Stephen

(57) **Abstract**

A surgical access device has an instrument seal. A wound protector is connectable to the instrument seal. The wound protector comprises a distal ring, a proximal ring, and a resilient sleeve extending between the distal and proximal rings. The diameter of the distal ring is adjustable between a retracted position and an expanded position.

## Description

### BACKGROUND

The present invention relates in general to surgical devices and procedures, and more particularly to access devices.

Surgical procedures are often used to treat and cure a wide range of diseases, conditions, and injuries. Surgery often requires access to internal tissue through open surgical procedures or endoscopic surgical procedures. The term "endoscopic" refers to all types of minimally invasive surgical procedures including laparoscopic and arthroscopic procedures. Endoscopic surgery has numerous advantages compared to traditional open surgical procedures, including reduced trauma, faster recovery, reduced risk of infection, and reduced scarring. Endoscopic surgery is often performed with an insufflatory fluid present within the body cavity, such as carbon dioxide or saline, to provide adequate space to perform the intended surgical procedures. The insufflated cavity is generally under pressure and is sometimes referred to as being in a state of pneumoperitoneum. Surgical access devices are often used to facilitate surgical manipulation of internal tissue while maintaining pneumoperitoneum. For example, trocars are often used to provide a port through which endoscopic surgical instruments are passed. Trocars generally have a sealing valve that prevents the insufflatory fluid from escaping while an instrument is positioned in the trocar. As a further example, hand access ports are also used during endoscopic surgery, sometimes referred to as hand assisted laparoscopic surgery ("HALS"). A hand access port will typically seal around a surgeon's hand or arm to prevent the insufflatory fluid from escaping while allowing the surgeon to manipulate tissue within the patient's body.

While surgical access devices are known, no one has previously made or used a surgical access device in accordance with the present invention.

### BRIEF DESCRIPTION OF DRAWINGS

While the specification concludes with claims which particularly point out and distinctly claim the invention, it is believed the invention will be better understood from the following description taken in conjunction with the accompanying drawings illustrating some non-limiting examples of the invention. Unless otherwise indicated, the figures are not necessarily drawn to scale, but rather to illustrate the principles of the invention.
Fig. 1 depicts a perspective view of a surgical access device;
Fig. 2 depicts an exploded view of Fig. 1 and additionally including an instrument seal;
Fig. 3 depicts a top plan view of Fig. 1;
Fig. 4 depicts a top plan view of an alternative surgical access device;
Fig. 5 depicts a partial cross-sectional view of an alternative surgical access device;
Fig. 6 depicts a cross-sectional view of the surgical access device Fig. 5 in its retracted position; and
Fig. 7 depicts a cross-sectional view of the surgical access device Fig. 5 in its expanded position.

### DETAILED DESCRIPTION

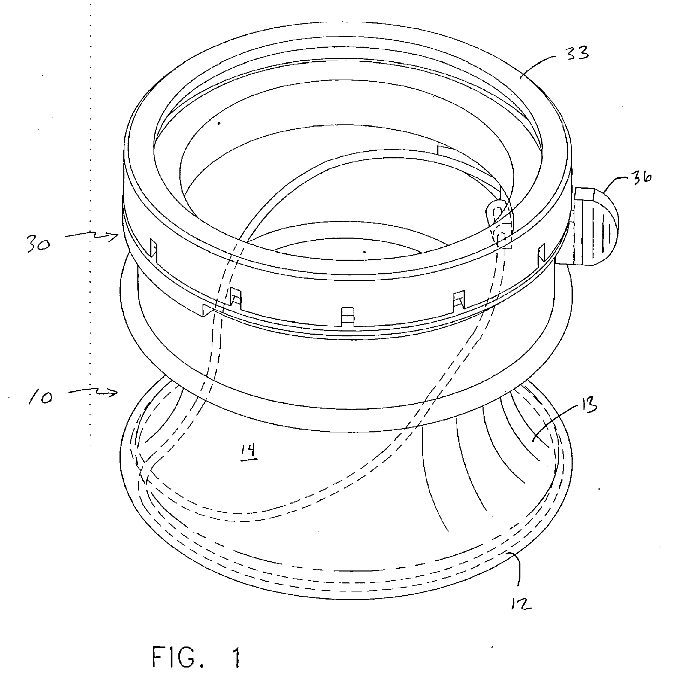

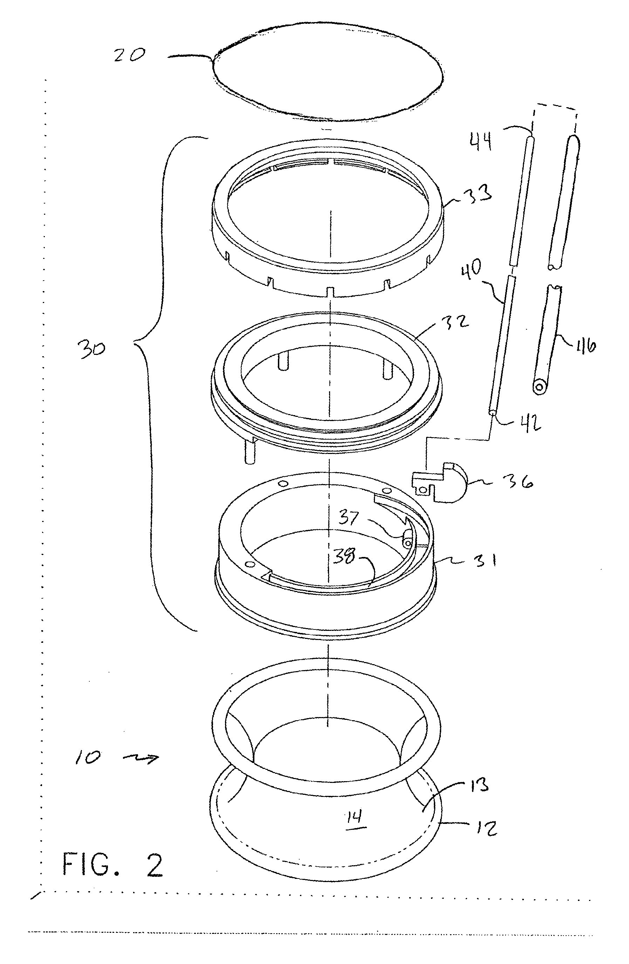

Figs. 1-4 illustrate an example of a surgical access device. The device includes a wound protector (10) and a housing (30) that may contain an instrument seal (20). The wound protector comprises a resilient sleeve (14). In this embodiment the sleeve (14) is a single layered tube of resilient material; however, a discontinuous sleeve or multi-layered sleeves are also possible. Without limitation, the sleeve (14) may be made from polyurethane-based elastomer, polyester-based elastomer, polyolefin-based elastomer, polyamide-based elastomer, silicone rubber, latex rubber, synthetic isoprene rubber, fluoropolymer-based elastomers, elastomeric copolymers of polyesters and siloxanes, polyurethanes and polysiloxanes, and other elastomeric co- or tri-polymers such as styrene-ethylene/butylene-styrene (S-EB-S), and the like. The sleeve (14) may be transparent, translucent, or opaque. As shown here, the sleeve (14) is fastened at its distal end to a hollow distal ring (12) using an adhesive or heat sealing techniques; however, alternative techniques may also be employed. The sleeve (14) is fastened at its proximal end to the proximal ring (32). The diameter and length of the sleeve (14) can vary depending on the application. For instance, the sleeve may be between 1 cm and 6 cm in length and between 1 cm and 10 cm in diameter; however, other lengths and diameters are also possible. The thickness of the sleeve (14) can also vary.

As an example of use in abdominal surgery, the wound protector (10) could be placed in an incision in the abdominal wall such that the distal ring (12) is adjacent the peritoneal surface and the ring (32) is adjacent the cutaneous surface. The proximal and distal rings (32, 16) are laterally disposed relative the incision to define a flange (13) that facilitates holding the wound protector (10) in the incision. In this embodiment the proximal ring (32) and distal ring (12) are circular; however, non-circular rings are also possible.

The retaining ring (33) attaches to the cap (32) to compress and retain therebetween an instrument seal (20. When an object such as a surgical instrument is placed through the access device, the seal (20) engages the object (30) and prevents insufflatory fluids from escaping from the surgical site through the device. The seal (20) in this embodiment is a diaphragm seal; however, the specific seal mechanism is not important to the present invention, so any number of other known instrument seals or HALS seals could also be used, including without limitation iris valves, multilayered seals, gel seals, foam seals, and the like.

The proximal ring (31) includes a channel (38) with a port that opens to the medial face of the ring (31). It is contemplated, however, that the port may also open to the lateral face. A slider (36) is positioned in the channel (38) and extends out the lateral face of the ring (31). The cap (32) attaches to the ring (31) to close the channel (38) and contain the slider (36) in the channel (38). The slider (36) can move in the channel (38) to change is angular position relative the ring (31). A detent mechanism between the slider (36) and channel (38) provides a locking feature so the slider (36) will retain its selected angular position in the channel (38).

A wire (40) includes a proximal end (42), a distal end (44), and a length extending between the proximal and distal ends. Without limitation, the wire (40) diameter may be between about 1 mm and about 7 mm. The wire can be made of a variety of different materials including nitinol, stainless steel, polymer wires such as polyimide, polyetherimides (PEI), polyaryletherketones such as PEEK (poly ether ether ketone), carbon or glass composites, and the like. The proximal end (42) of the wire (40) is positioned in the channel (38) and is attached to the slider (36). The wire (40) extends out of port and continues medially within the sleeve (14) to the distal ring (12). The wire (40) enters the hollow distal ring (12) and continues substantially about its circumference until it exits from the distal ring (12) in the opposite direction from which it entered. The wire (40) continues back medially within the sleeve (14) to the proximal ring (31) where it is attached to the boss (37). Accordingly, the wire in this embodiment takes a double looped geometry. One loop is in the plane of the distal ring (12) while the second loop extends proximally at an oblique angle relative the plane.

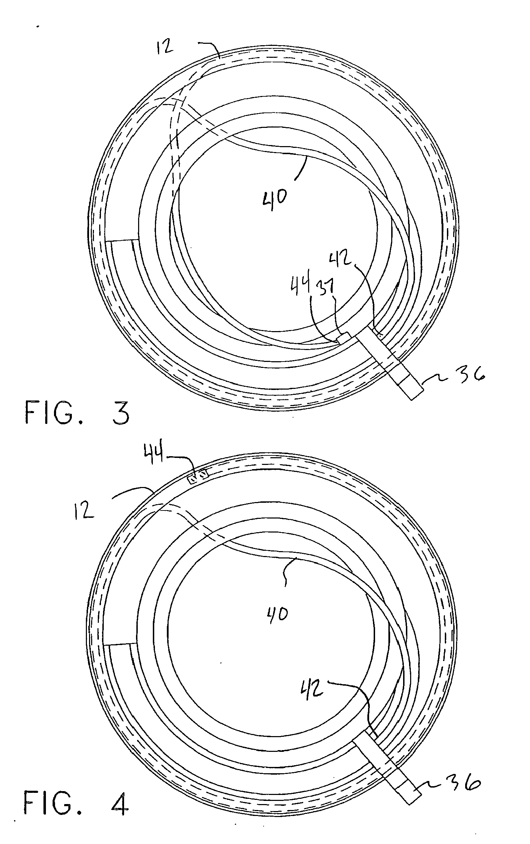

Fig. 4 illustrates an alternative wire (40) configuration. The proximal end (42) of the wire (40) is positioned in the channel (38) and is attached to the slider (36). The wire (40) extends out of the medial facing port and continues medially within the sleeve (14) to the distal ring (12). The wire (40) enters the hollow distal ring (12) and continues substantially about its circumference. The distal end (44) is then attached to the distal ring (20). Accordingly, the wire in this embodiment takes a one and one-half loop geometry. One loop is in the plane of the distal ring (12) while a partial loop extends proximally at an oblique angle relative the plane.

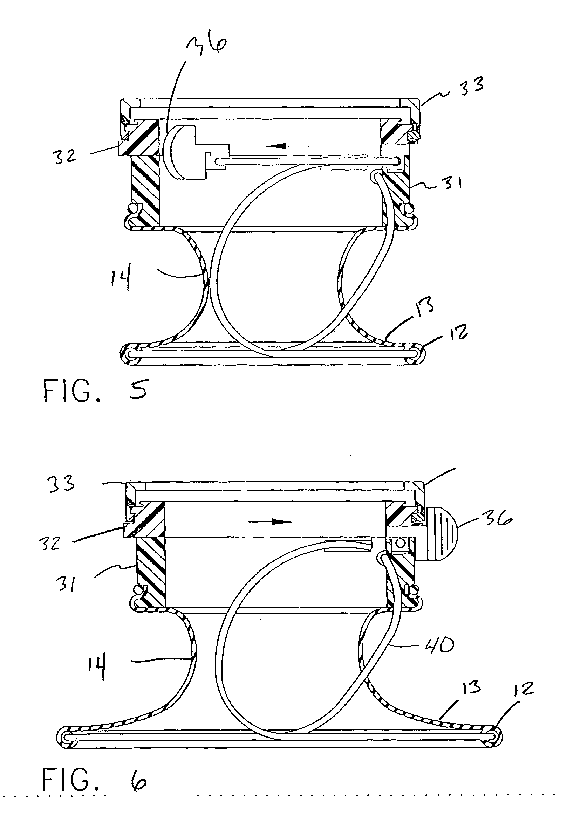

Figs. 5-6 illustrate a device in use. As shown in Fig. 5, the slider (36) is rotated clockwise-most position, thus pulling the wire (40) into the channel (38). Accordingly, the length of wire (40) in the distal ring (12) is shortened. The wire (40) is designed to slide in the distal ring (12). Optionally, a lubricant may be added in the distal ring (12) to reduce friction. Since the distal ring (12) is resilient, the shortening of the wire (40) will retract the diameter of the distal ring (12). This retracted distal ring (12) helps facilitate placement of the wound protector (10) into the incision. As shown in Fig. 6, the slider (36) is rotated counter-clockwise-most position, thus pushing the wire (40) out of the channel (38). Accordingly, the length of wire (40) in the distal ring (12) is lengthened. Since the distal ring (12) is resilient, the lengthening of the wire (40) will expand the diameter of the distal ring (12). This expanded distal ring (12) helps increases the size of the flange (13), thus improving the fixation of the wound protector (10) in the incision, and may also facilitate retraction of the incision by the sleeve (14). Optionally, the portion of the wire (40) outside the proximal ring (31) and distal ring (12) will be position and sheath (46) to create a bowden-type cable configuration so the wire (40) can better transmit compressive and tensile loads to the distal ring (12).

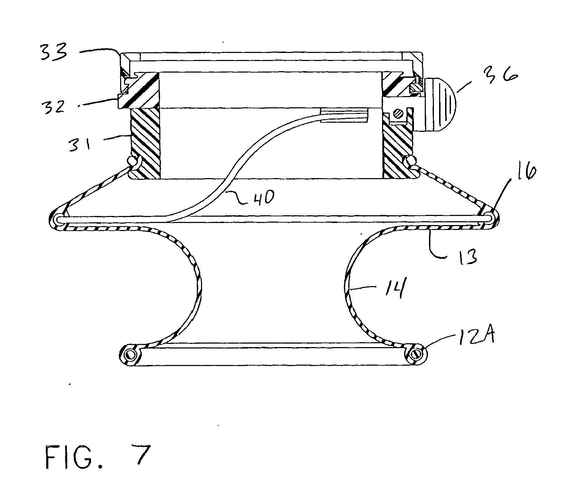

Fig. 7 illustrates another embodiment of an access device. The distal ring (12A) is a fixed diameter ring. The wire (40) is positioned in an intermediate ring (16), which would be adjacent the cutaneous surface of the abdominal wall. After the distal ring (12A) is positioned through an incision, the slider (36) may be adjusted to expand the intermediate ring (16) to facilitate retraction of the incision by the sleeve (14).

Preferably, the devices described above will be processed before surgery. First, a new or used device is obtained and if necessary cleaned. The device can then be sterilized. In one sterilization technique the access device is placed in a closed and sealed container, such as a plastic or TYVEK bag. Optionally, the device can be bundled in the container as a kit with other components, including one or more of the following: a wound protector, access port, a tube of lubricant, a marker, an incision template or scale, an instruction sheet, and the like. The container and device, as well as any other components, are then placed in a field of radiation that can penetrate the container, such as gamma radiation, x-rays, or high-energy electrons. The radiation kills bacteria on the device and in the container. The sterilized device can then be stored in the sterile container. The sealed container keeps the device sterile until it is opened in the medical facility.

Having shown and described various embodiments and examples of the present invention, further adaptations of the methods and devices described herein can be accomplished by appropriate modifications by one of ordinary skill in the art without departing from the scope of the present invention. Several of such potential modifications have been mentioned, and others will be apparent to those skilled in the art. For instance, the specific materials, dimensions, and the scale of drawings will be understood to be non-limiting examples. In addition, the foregoing teachings could be implemented for HALS procedures. Accordingly, the scope of the present invention should be considered in terms of the following claims and is understood not to be limited to the details of structure, materials, or acts shown and described in the specification and drawings.

## Claims

1. A surgical access device, comprising:
a) an instrument seal;
b) a wound protector connectable to the instrument seal, the wound protector comprising a distal ring, a proximal ring, and a resilient sleeve extending between the distal and proximal rings;
c) a wire comprising a proximal end, a distal end, and a length extending between the proximal and distal ends, wherein at least a portion of the wire length is slideably positioned in the distal ring and the portion of the wire length in the distal ring is variable to adjust the diameter of the distal ring between a retracted position and an expanded position.

2. A surgical access device of claim 1, wherein the proximal end of the wire is positioned in a channel in the proximal ring.

3. The surgical device of claim 2, wherein the channel includes a medially facing port.

4. A surgical access device of claim 2, further comprising a slider positioned in the channel, wherein the proximal end of the wire is attached to the slider.

5. A surgical access device of claim 1, wherein the distal end of the wire is attached to the proximal ring.

6. The surgical access device of claim 5, wherein the wire has a double loop configuration.

7. The surgical access device of claim 6, wherein one loop is in the plane of the distal ring, and the other loop is at an oblique angle relative the plane.

8. A surgical access device of claim 1, wherein the distal end of the wire is attached to the distal ring.

9. A surgical access device of claim 3, further comprising a sheath positioned between the port and the distal ring, the sheath receiving a portion of the wire length.

10. A surgical access device, comprising:
a) an instrument seal;
b) a wound protector connectable to the instrument seal, the wound protector comprising a distal ring, a proximal ring, and a resilient sleeve extending between the distal and proximal rings, wherein the diameter of the distal ring is adjustable between a retracted position and an expanded position.

11. A surgical access device, comprising:
a) an instrument seal;
b) a wound protector connectable to the instrument seal, the wound protector comprising a distal ring, a proximal ring, a resilient sleeve extending between the distal and proximal rings, and an intermediate ring attached to the sleeve intermediate the proximal and distal rings;
c) a wire comprising a proximal end, a distal end, and a length extending between the proximal and distal ends, wherein at least a portion of the wire length is slideably positioned in the intermediate ring and the portion of the wire length in the intermediate ring is variable to adjust the diameter of the intermediate ring between a retracted position and an expanded position.
